Europäisches Patentamt

European Patent Office

Office européen des brevets

(19)

(11) EP 0 844 003 A1

(12) EUROPEAN PATENT APPLICATION
published in accordance with Art. 158(3) EPC

(43) Date of publication:
27.05.1998 Bulletin 1998/22

(51) Int. Cl.⁶: **A61K 38/21**

(86) International application number:
PCT/JP96/02064

(21) Application number: 96924199.1

(22) Date of filing: 23.07.1996

(87) International publication number:
WO 97/05897 (20.02.1997 Gazette 1997/09)

(84) Designated Contracting States:
AT BE CH DE DK ES FI FR GB GR IE IT LI LU MC
NL PT SE

(30) Priority: 08.08.1995 JP 202581/95

(71) Applicant:
OTSUKA PHARMACEUTICAL CO., LTD.
Chiyoda-ku Tokyo 101 (JP)

(72) Inventors:
• YAMAMOTO, Norimi
Otsuka Pharmaceutical Co., Ltd.
Tokushima 771-01 (JP)

• SHIBAMORI, Masahumi
Otsuka Pharmaceutical Co., Ltd
Tokuushima 771-01 (JP)
• KIKUCHI, Mikio
Otsuka Pharmaceutical Co., Ltd.
Tokushima 771-01 (JP)

(74) Representative:
Hansen, Bernd, Dr. Dipl.-Chem. et al
Hoffmann Eitle,
Patent- und Rechtsanwälte,
Arabellastrasse 4
81925 München (DE)

(54) **REMEDY FOR COXSACKIE VIRUS B INFECTION**

(57)   A remedy for Coxsackie virus B infection for which there has been no efficacious therapeutic means, which contains γ interferon as the active ingredient.

Fig. 2

EP 0 844 003 A1

Printed by Xerox (UK) Business Services
2.16.3/3.4

**Description**

TECHNICAL FIELD

The present invention relates to a therapeutic composition for diseases caused by coxsackievirus B infection which comprises gamma-interferon (γ-IFN) as an active ingredient.

BACKGROUND ART

Interferons (IFNs) are currently classified into three groups, namely alpha, beta and gamma. Among them, gamma interferon (γ-IFN) is an IFN produced upon stimulation of sensitized T lymphocytes by antigen binding or upon stimulation of unsensitized T lymphocytes by a mitogen or the like and is also called immune interferon. Said γ-IFN is composed of 146 amino acid residues. It is unstable against heat (56°C) and acids (pH 2) and, upon SDS-polyacrylamide gel electrophoresis (SDS-PAGE), it is separated into two fractions, one with a molecular weight of 25,000 and the other with a molecular weight of 20,000. Its amino acid sequence and the corresponding base sequence distinctly differ from those of alpha or beta group interferons.

In 1981, Gray et al. succeeded in introducing the human γ-IFN gene into Escherichia coli and causing expression of said gene in said host and determined the gene structure and the whole amino acid sequence of the protein, revealing that it has a molecular weight of 17,000 and that the gamma group comprises only one species [Gray, P. W. et al., Nature, 295, 503 (1982)]. Interferons were first discovered as antiviral substances elaborated by cells in response to stimulation by viruses etc. [Nagano, Y. & Kojima, Y., C. R. Soc. Biol., 148, 1700 (1954); Isaacs, A. & Lindenmann, J., Proc. Roy. Soc. B., 147, 258 (1984)]. Since then, it has been revealed that they have antitumor activity [Ida, N., "Interferon no Kagaku (Science of Interferons)", 11, Kodansha (1985), p. 44] as well as various immunoactivating and immunopotentiating activities such as killer T cell potentiating activity, natural killer activity potentiating activity and ADCC activity potentiating activity.

Viruses against which IFNs, in particular γ-IFN, show antiviral activity reportedly include herpes simplex virus [Martin, J. B. et al., J. Infect. Dis., 166, 1401-1403 (1992)], hepatitis B virus [Mora, C. V., et al., J. Interferon Res., 6, Suppl. 1, 133 (1986)], etc. No relevant report is available as yet, however, on coxsackievirus B.

Currently, in the field of clinical medicine, IFNs are used in the treatment of renal cell carcinoma, malignant melanoma, chronic hepatitis type C, chronic hepatitis type B, chronic myelocytic leukemia, multiple myeloma, hairy cell leukemia, rheumatism, etc. γ-IFN is used particularly in the treatment of renal cell carcinoma and malignant melanoma among the diseases mentioned above. Furthermore, an attempt to use γ-IFN against mycosis fungoides has also been made.

The above-mentioned three groups of IFN differ in biological activity. All IFNs potentiate class I MHC antigen expression. On the other hand, the potentiation of class II MHC antigen expression is not found with the alpha and beta groups but observed only with the gamma group. As regards other biological activities in which the immune system is involved, the gamma group differs in several respects from the alpha and beta groups; for instance, the gamma group shows stronger activity than the alpha and beta groups.

On the other hand, coxsackievirus belongs to a group of the genus Enterovirus of the family Picornavidae. It is a spherical (regular icosahedron), nonenveloped virus with a diameter of about 25 to 30 nm. From the antigenicity viewpoint, it is classified into two types (A and B) and each further includes a number of serotypes. As typical examples, there may be mentioned coxsackievirus A1 to 24 and coxsackievirus B1 to 6, which are causative of viral gastroenteritis. The type A virus is also causative of human herpangina and hand-foot-and-mouth disease, while the type B virus is a source of infection of proliferative glomerulo-nephritis and also is causative of epidemic pleurodynia. Furthermore, both types are causative of aseptic meningitis, myocarditis, pericarditis and acute juvenile diabetes mellitus. In particular, in acute juvenile diabetes mellitus, it is said that a certain variant of coxsackievirus B4 directly destroys pancreatic Langerhans' islands and induces diabetes mellitus independently of autoimmune responses. In the diagnostic field, a method of establishing viral infection of myocardial biopsy samples from chronic myocarditis patients has been attempted using the coxsackievirus cDNA.

In the treatment of the above-mentioned myocarditis, in addition to rest and feeding, anti-inflammatory agents, for example aspirin, and diuretics are administered in mild cases. Furthermore, regarding myocarditis as an event of immunological abnormality, it has been suggested that steroidal agents or immuno-suppressants such as cyclosporin be administered. However, it has been reported that such agents experimentally may rather cause aggravation. Thus, they should be administered only in severe cases when other therapeutic means are ineffective and with caution.

Meanwhile, there are several reports dealing with the relationship between viral myocarditis and IFNs. Lutton et al. report that, in a mouse viral myocarditis model, murine beta IFN (β-IFN) indeed prevented viral infection in groups in which β-IFN administration was started at 24 hours before or immediately after inoculation with coxsackievirus B3. However, the administration was begun on 3 days after inoculation, said administration was rather harmful [Lutton, C.

W. & Gauntt, C. J., J. Interferon Res. 5, 137-146 (1985)]. Further, Matsumori et al. investigated whether recombinant human IFN-α A/D administered subcutaneously to coxsackievirus B3-infected mice at a dose of $10^4$ U/g mouse on the day before virus inoculation and immediately after inoculation could prevent viral replication in the virus-infected mouse heart and improve the histopathological picture of the heart. They confirmed the inhibition of viral replication and, as for the amelioration of histopathological heart picture, they report that said IFN was effective in inhibition of inflammatory cell infiltration as well as myocardial cell necrosis [Matsumori, A., et al. Am. Heart J., 115, 1229-1232 (1988)].

Furthermore, Okada et al. report that, in an in vitro test in which rivabirin and recombinant human IFN-α A/D were combinedly used, recombinant human IFN-α A/D, even alone, showed antiviral activity against coxsackievirus B3, the $ED_{50}$ value being 42.1 IU/ml [Okada, I., et al., J. Lab. Clin. Med., 120, 569-573 (1992)].

On the other hand, Seko et al. administered γ-IFN to mice infected with coxsackievirus B3 and investigated MHC antigen expression in myocardial cells by immunofluorescence and Northern blot analyses. Reporting that class I MHC antigen expression and very low level class II MHC antigen expression were observed, they suggest that class I MHC antigen expression induces interaction between myocardial cells and T lymphocytes and in particular that cytotoxic T lymphocytes play a partial role in heart injury induced by viral infection [Seko, Y., Circulation Res., 67 (2), 360-367 (1990)].

Further, Seko et al. administered γ-IFN to coxsackievirus B3-infected mice as described in the above-cited reference and confirmed ICAM-1 antigen expression in myocardial cells in an acute myocarditis model. According to their report, anti-ICAM-1 antibody treatment inhibited coxsackievirus B3-induced myocarditis [Seko, Y., et al., J. Clin. Invest., 91, 1327-1336 (1993)].

Smith et al. report that administration of anti-γ-IFN antibody to A/J mice in which myocarditis had been induced by immunization with myosin resulted in statistically significant increases in the severity of myocarditis as compared with rat immunoglobulin G and control groups [Smith, S. C. & Allen, P. M., Circulation Research 70 (4), 856-863 (1992)].

Furthermore, Kipshidze et al. detected antibodies to coxsackievirus B and influenza virus in patients with viral myocarditis eventually succumbing to death. They reported decreased α- and, in particular γ-IFN activities [Kipshidze, N. N., et al., Ter. Arkh., 60 (11), 43-46 (1988)].

In addition, Durand et al. discuss the relationship between the level of γ-IFN produced by monocytes in patients with coxsackievirus B4 infection-induced myocarditis or acute rhabdomyolysis and the clinical findings [Durand, J. M. & Soubeyrand, J., Rev. Med. Interne, 14/10, 1128 (1993)].

## DISCLOSURE OF THE INVENTION

Although reports are available about the relationship between coxsackievirus B myocarditis and γ-IFN, as mentioned above, there are no reports about an antiviral effect produced by administration of γ-IFN to patients with coxsackievirus B infection or about alleviation or remission of various symptoms of viral infection as attained by administration of γ-IFN. Thus, it remains unkhown whether administration of γ-IFN is effective against myocarditis caused by coxsackievirus B infection, in particular coxsackievirus B3 infection.

Accordingly, the present invention has for its object to provide a therapeutic composition for coxsackievirus B infections, particularly the myocarditis and pericarditis associated with said virus, which composition comprises γ-IFN as an active ingredient.

The present inventors made intensive investigations to accomplish the above object and found that when recombinant γ-IFN is administered against the above-mentioned infectious diseases, significantly higher survival rates can be obtained as compared with infected controls, with inhibitory effects on the progress of myocardial lesions and significant decreases in endocardial virus count. Based on these findings, they have now completed the present invention.

Thus, the present invention provides a therapeutic composition for diseases caused by coxsackievirus B which comprises γ-IFN as an active ingredient, further a therapeutic composition for diseases caused by coxsackievirus B where the coxsackievirus B infection occurs in the myocardium and/or pericardium which composition comprises γ-IFN as an active ingredient, and further a therapeutic composition for diseases caused by coxsackievirus B where the coxsackievirus B infection is coxsackievirus B3 infection which composition comprises γ-IFN as an active ingredient.

The γ-IFN to be used as an active ingredient in the therapeutic composition of the present invention for diseases caused by coxsackievirus B infection may be either natural γ-IFN or a genetically engineered one (recombinant) provided that it has γ-IFN activity. Generally, natural γ-IFN is produced by a method comprising collecting human leukocytes or culturing ordinary human cells to obtain T cells of an established cell line and then inducing the cells to produce γ-IFN using PHA, ConA or the like as an inducer [Nathan, I. et al., Nature, 292, 842 (1982); Matuyama, M., et al., J. Immunol., 129, 501 (1982)].

For carrying out the above-mentioned method, it is in many cases difficult to obtain cells in large quantities from the technical and cost viewpoints. For lower cost cell growth, the so-called hamster method has been developed (see Japanese Patent Publication 63-1296 and Japanese Patent Publication 56-54158). According to said method, T cell multiplication is caused by transplanting established line T cells subcutaneously to the back of young hamsters given an immunosuppressant in advance. After growth of the tumor lump on the hamster back to attain a certain size, the tumor lump is excised and minced, a cell suspension is prepared by dispersing the cells, the thus-prepared T cells are induced to produce γ-IFN using PHA. The γ-IFN obtained by said method has the same carbohydrate chain as that of the natural type.

In addition, for producing γ-IFN stably and at low cost, a method is also known which comprises cultivating a microorganism with a human γ-IFN gene prepared by the recombinant DNA technology and introduced into said microorganism [Gray, P., et al., Nature, 295, 501 (1982)]. The γ-IFN obtained by said method has no carbohydrate chain, however.

Further known, as a method of producing a polypeptide having human γ-IFN activity, is the method disclosed in Japanese Patent Publication 07-45516 and, in Japanese Patent Publication 07-45515, there is disclosed a method of producing a novel human γ-IFN. The IFNs obtained by these methods all can be used as the active ingredient of the composition according to the present invention. The one produced by the hamster method is particularly preferred in carrying out the present invention.

In clinically using the therapeutic composition of the present invention for coxsackievirus B infection, the daily effective amount for each adult can be selected within a broad range. Generally, when human γ-IFN is used as the active ingredient, said amount is desirably selected within the range of about 100,000 to 2,000,000 international units per body per day.

The therapeutic composition of the present invention for coxsackievirus B infection which comprises human γ-IFN as an active ingredient may be used in combination with another therapeutic composition for coxsackievirus B infection or may further contain the active ingredient compound of such other composition. As examples of such other active ingredient compound, there may be mentioned those that are used for coxsackievirus B myocarditis, such as aspirin, steroids, cyclosporin and the like immunosuppressants, among others. Such combined use is expected to produce synergistic effects, making it possible to reduce the amounts of both drugs to be combined and reduce the side effects of the drugs.

Similarly, the therapeutic composition of the present invention may also contain α-IFN or β-IFN or both. It is also possible to use the therapeutic composition of the present invention in combination with a therapeutic preparation containing such other IFN. Such combined use makes it possible to reduce the dose of one or both IFNs and reduce the side effects.

For the other IFNs suited for the above-mentioned combined use, for example natural α-type human IFN and β-type human IFN, large-scale production methods have already been established. Thus, known as a method of producing natural α-type human IFN on a large scale is the method comprising using, among human acute leukemia cell lines, cells capable of producing high potency α-IFN by viral induction (see, for example, Japanese Kokai Tokkyo Koho 54-98307; Japanese Kokai Tokkyo Koho 55-47629; Japanese Kokai Tokkyo Koho 55-62024). As regards the production of human β-IFN using the recombinant DNA technology, there may be mentioned the methods described in Laid-open European Patent Application 81-28033, Laid-open European Patent Application 81-321134, Laid-open European Patent Application 81-34307 and Belgian Patent 81-837397, among others.

The above-mentioned other therapeutic agent and/or other IFN preparations need not be administered always simultaneously with the therapeutic composition of the present invention but may be administered separately within the period which the effect of the therapeutic composition of the present invention can be expected.

In the above-mentioned case of combined administration of IFNs, γ-IFN, for example human γ-IFN, as the active ingredient of the therapeutic composition of the present invention is desirably administered at a general daily dose of about 100,000 to 2,000,000 international units per body, while α- and/or β-IFN is desirably used in combination with γ-IFN at a general daily dose of about 100,000 to 5,000,000 international units per body. When α- and/or β-IFN and γ-IFN are combinedly used, the dose ratio α- and/or β-IFN:γ-IFN is preferably selected within the range of 1:1 to 10,000:1, preferably 1:1 to 1,000:1, more preferably 5:1 to 100:1 (units).

The therapeutic composition of the present invention for coxsackievirus B infection can be used, according to the purpose of its use, in various dosage forms which are conventional in this field of art. In particular, in the practice of the present invention, it is also possible to increase the stability of the active ingredient γ-IFN by using human serum albumin and/or a carbohydrate and a surfactant in producing the dosages.

The carbohydrate mentioned above is not limited to any particular species but use may be made of monosaccharides such as glucose, mannose, galactose and fructose, sugar alcohols such as mannitol, inositol and xylitol, disaccharides such as sucrose, maltose and lactose, polysaccharides such as dextran and hydroxypropylstarch, and so forth. These may be used either singly or in combination as a mixture of two or more. Particularly preferred among them are sucrose, maltose, mannitol, inositol, dextran and the like.

The surfactant is not limited to any particular species, either. Ionic and/or nonionic surfactants may generally be used. Among them, such surfactants as polyoxyethyleneglycolsorbitan alkyl esters, polyoxyethylene alkyl ethers, sorbitan monoacyl esters, fatty acid glycerides and the like are preferred.

The carbohydrate mentioned above is suitably added at an addition level of about 0.1 mg or more, preferably about 1 to 100 mg, per microgram (μg) of the very γ-IFN or a derivative thereof such as mentioned above (hereinafter collectively referred to as "γ-IFN active substance") and the surfactant is suitably added at a level of about 0.0001 mg or more, preferably about 0.001 to 0.1 mg, per microgram of the γ-IFN active substance. Human serum albumin is suitably added at a level of about 0.001 mg or more, preferably about 0.01 to 10 mg, per microgram of the γ-IFN active substance.

The therapeutic composition of the present invention for coxsackievirus B infection may of the same kind as the conventional pharmaceutical compositions of this kind. Thus, one or more other pharmacologically active ingredients or common ingredients in pharmaceutical preparations production may be incorporated as desired.

Particularly, for further increasing the stability of the γ-IFN active substance, common sulfur-containing reducing agents are preferred as the other ingredients that can be incorporated in the therapeutic composition of the present invention for coxsackievirus B infection. Specific and preferred examples of said sulfur-containing reducing agents are such relatively mild reducing agents as cysteine, N-acetylhomocysteine, thioctic acid, thioglycolic acid, salts of these, sodium thiosulfate, thiolactic acid, dithiothreitol, glutathione and the like. These may be used singly or in combination (two or more). The level of addition of these is not particularly limited but is suitably about 0.001 mg or more, preferably about 0.01 to 10 mg (when two or more are used combinedly, the total amount), per microgram of the γ-IFN active substance.

It is also appropriate that the therapeutic composition of the present invention for coxsackievirus B infection be given a stable isotonic preparation form by isotonizing with a buffer. As typical examples of the buffer that can be used for this purpose, there may be mentioned various buffer solutions having a pH of about 4 to 8, preferably about 5 to 6, such as citric acid-sodium citrate, citric acid-sodium phosphate, acetic acid-sodium acetate and citric acid-sodium borate buffers.

The therapeutic composition of the present invention for coxsackievirus B infection is generally prepared in the form of a pharmaceutical preparation, for example by mixing a pharmacologically effective amount of the γ-IFN active substance, together with one or more specific ingredients such as mentioned above, with an appropriate carrier for pharmaceutical preparation production. Usable as said pharmaceutical carrier are those excipients or diluents that are generally used in producing pharmaceutical preparations according to the form of use, inclusive of fillers, extenders, binders, wetting agents, disintegrators, etc. The form of the pharmaceutical preparation is not limited to any particular one provided that it contains the active ingredient efficiently. Said form thus includes solid forms such as tablets, powders, granules and pills, as well as injection forms such as solutions, suspensions and emulsions. These may be in the form of dried products which can be converted to liquid forms prior to use by addition of an appropriate carrier. The pharmaceutical preparations mentioned above can all be produced by the conventional methods.

The thus-obtained pharmaceutical preparation is administered by an appropriate route according to the form thereof. Thus, for example, when it is an injectable one, it may be administered intravenously, intramuscularly, subcutaneously, intradermally or intraperitoneally. When it is a solid form preparation, it may be administered orally, rectally or via the intestine. When it is in the form of a solution, suspension or powder, it may be used as an inhalant or a nasal preparation as well. Said administration may be conducted once a day or dividedly 3 to 4 times a day.

Administration of the therapeutic composition of the present invention for coxsackievirus B infection, which comprises γ-IFN as an active ingredient produces an inhibitory effect on the progress of myocardial lesions and causes significant decreases in endocardial virus count in coxsackievirus B infection, in particular in myocarditis caused by said viral infection, giving a survival rate significantly higher as compared with infected controls. Thus, said composition is useful as a therapeutic composition for coxsackievirus B infection.

BRIEF DESCRIPTION OF THE DRAWINGS

Fig. 1 graphically shows the inhibitory effect on the body weight loss as produced in coxsackievirus B3-infected mice by intratracheal administration of γ-IFN.

Fig. 2 graphically shows the change in survival rate and the life span-prolonging effect as produced in coxsackievirus B3-infected mice by intratracheal administration of γ-IFN.

Fig. 3 graphically shows the inhibitory effect on the endocardial viral replication as produced in coxsackievirus B3-infected mice by intratracheal administration of γ-IFN.

Fig. 4 graphically shows the comparison of inhibitory effect on the endocardial viral replication as produced in coxsackievirus B3-infected mice by intratracheal administration of γ-IFN or α/β-IFN.

BEST MODES FOR CARRYING OUT THE INVENTION

The following reference examples and working examples are further illustrative of the present invention.

Reference Example 1 [Preparation of γ-IFN]

γ-IFN was prepared according to Japanese Patent Publication 63-1296 as follows.

## (1) Cell multiplication

BALL-1 cells were cultured in RPMI-1640 medium (pH 7.2) containing 20% FCS (fetal calf serum) at 37°C. The cells obtained were washed with serum-free RPMI-1640 medium (pH 7.2) and then suspended in the same medium to a concentration of about $1 \times 10^6$ cells/ml. The BALL-1 cells prepared as above were used to subcutaneously inoculate newborn hamsters given rabbit antiserum in advance for suppressing immune responses. The animals were fed as usual for about 3 weeks. Then, the BALL-1 cell tumor lumps grown subcutaneously were excised, minced and suspended in trypsin-containing physiological saline to disperse cells.

The cells obtained were washed with serum-free RPMI-1640 medium and suspended in the same medium containing 10% FCS to a concentration of about $1 \times 10^6$ cells/ml for submission to the subsequent step of $\gamma$-IFN production.

## (2) Production of $\gamma$-IFN

To the BALL-1 cells obtained as mentioned above in (1) and suspended in 10% FCS was added 100 ng of lipopolysaccharide (LPS) as an inducer. Then, induced production of $\gamma$-IFN was caused by culturing at 37°C for 3 days.

After cultivation, cells were removed by centrifugation and the filtrate was concentrated by ultrafiltration. The concentrate was purified using a monoclonal antibody column. The sample obtained had a specific activity of $7.35 \times 10^6$ IU/mg protein.

## Example 1 Effect of $\gamma$-IFN in coxsackievirus B myocarditis model

### (1) Construction of viral myocarditis model

Using Eagle's MEM medium supplemented with 10% fetal calf serum (hereinafter referred to as 10% E-MEM), the Nancy strain of coxsackievirus B3 (ATCC VR-30, SM/2+, MkK/14, LLC-Mk2/3 generation-subcultured strain, obtained from the American Type Culture Collection: ATCC) was further subcultured in HeLa (3 passages) and in C3H/He mice (12 passages) at the laboratory of Otsuka Pharmaceutical Co. The heart of each symptomatic mouse was excised and treated in 10% E-MEM to give a 10% emulsion. The emulsion was centrifuged and the supernatant was stored in an ultralow temperature vessel at -80°C (stock strain, 10% infected mouse heart emulsion supernatant).

The above stock strain was diluted to $1.0 \times 10^6$ pfu/ml. Three-week-old male C3H/He slc mice were intra-peritoneally inoculated with the above virus-containing dilution at an inoculum size of 0.1 ml. The above-mentioned experimental mice were purchased from the firm SLC. The mice purchased were weighed on the day next to the day of arrival and grouped by weight into 7 groups each comprising 20 animals using the TRIM grouping soft (compiler edition data processing system) and were thus grouped into uninfected, infected control and INF-given groups for the subsequent experiment.

### (2) Preparation of murine $\gamma$-IFN

The murine $\gamma$-IFN used was recombinant murine $\gamma$-IFN (hereinafter referred to as m-$\gamma$IFN) obtained from Hayashibara Biochemical Laboratories, Okayama Prefecture (lot number: 004003). The biological activity of said m-$\gamma$IFN was $6.52 \times 10^5$ IU/ml ($6.27 \times 10^5$ IU/mg protein).

The biological activity of an already known m-$\gamma$IFN was $7.5 \times 10^5$ IU/mg protein [Yoshida, T. et al., katei Igaku (Family Medicine), 8, Suppl. 2, 19-22 (1992)].

### (3) Preparation of murine $\alpha/\beta$-IFN

The murine $\alpha/\beta$-IFN used were natural murine $\alpha/\beta$-IFN (hereinafter referred to as m-$\alpha/\beta$IFN) obtained from Hayashibara Biochemical Laboratories, Okayama Prefecture (lot number: 005). The biological activity of said m-$\alpha/\beta$IFN as determined based on CPE using the combination of L929 cells and VSV was $6.95 \times 10^7$ IU/ml ($3.56 \times 10^7$ IU/mg protein).

### (4) Administration of each IFN to virus-infected mice

The concentration of m-$\gamma$IFN was adjusted to $5 \times 10^5$ IU/ml using physiological saline. This was further diluted to $5 \times 10^3$ or $5 \times 10^4$ IU/ml using 0.01% mouse albumin-physiological saline.

The concentration of m-$\alpha/\beta$IFN was adjusted to $5 \times 10^3$, $5 \times 10^4$ or $5 \times 10^5$ IU/ml using 0.01% mouse albumin-physiological saline.

After inoculation with the virus as in (1), mice were anesthetized with 0.1 ml/mouse of a 2-fold dilution of Ketalar 50 (Sankyo-Zoki Kabushiki Kaisha) in saline as injected into the hind-limb femoral muscle of each mouse, and using a microsyringe (Microliter #710, Hamilton), 20 μl each of m-γIFN and m-α/βIFN solutions of the above-mentioned concentrations were respectively administered into the trachea via the larynx under Mimi-Pick 33 (Pyro Electric Company) illumination for 4 consecutive days following inoculation.

For evaluating the life span-prolonging effect, the survival times in days were recorded up to day 21 after inoculation, the survival rates were tested by the Kaplan-Meier method, and the homogeneity of the survival curves was tested by generalized Wilcoxon test. However, to adjust for multiplicity, Bonferroni's correction was made.

For the evaluation of dose dependence, Cochran-Armitage trend test (C-A test) was performed using the numbers of surviving animals and dead animals on day 21 after inoculation.

For the evaluation of antiviral effect, the heart was excised 7 days after inoculation, emulsified in 10% E-MEM, and monolayer-cultured. By the plaque assay using FL cells, the endocardial virus population was estimated. The effective dose was estimated by one-way analysis of variance (Anova) followed by the two-tailed Dunnett's test.

The pathological picture-improving effect was evaluated as follows. The heart was excised 7 days after inoculation and pathological specimens of the heart were prepared. The lesion scores were arrived at as follows. According to the percentage of the lesion based on the total area of the heart (ventricular) section, the case in which the percentage of the lesion was less than 25% was scored as +1; 25~50% as +2; 50-75% as +3; and more than 75% as +4. The cardiac pathology index was obtained by dividing the total score by the number of specimens examined. For a further evaluation of dose dependence, lesion scores were graded into ≤+3 and ≥+4 or ≤+2 and ≥+3 and C-A test was made.

The effective dose was estimated by Mann-Whitney U test. The evaluation was made after Bonferroni's correction for multiplicity.

In all tests, the 5% level of significance was used.

The results obtained above, together with the results obtained for the group given 0.01% mouse albumin alone (infected control group), are shown below.

a) Mouse body weight change

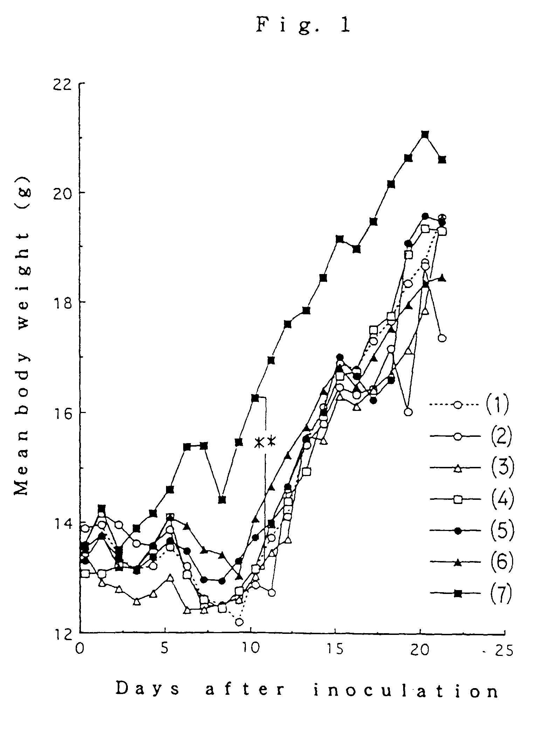

The results are shown in Fig. 1 (ordinate: mean body weight (g); abscissa: number of days after inoculation).

In the figure, the zigzag lines respectively corresponds to the following dosed groups. In the figures appearing later, too, the respective dosed groups are indicated by the same symbols as used here.

(1) - infected control group given 0.01% mouse albumin alone (n = 14)
(2) - 100 IU m-α/βIFN-dosed group (n = 7)
(3) - 1,000 IU m-α/βIFN-dosed group (n = 9)
(4) - 10,000 IU m-α/βIFN-dosed group (n = 9)
(5) - 100 IU m-γIFN-dosed group (n = 16)
(6) - 1,000 IU m-γIFN-dosed group (n = 15)
(7) - 10,000 IU m-γIFN-dosed group (n = 12)

As seen in Fig. 1, dose dependence was observed in the groups intratracheally given m-γIFN (linearity: p = 0.005, deviation from the line: p = 0.2418). Further, in the 10,000 IU/mouse group (graph (7)) alone, body weight loss was significantly inhibited at the 1% level, presumably due to m-γIFN administration.

b) Survival rate and life span-prolonging effect

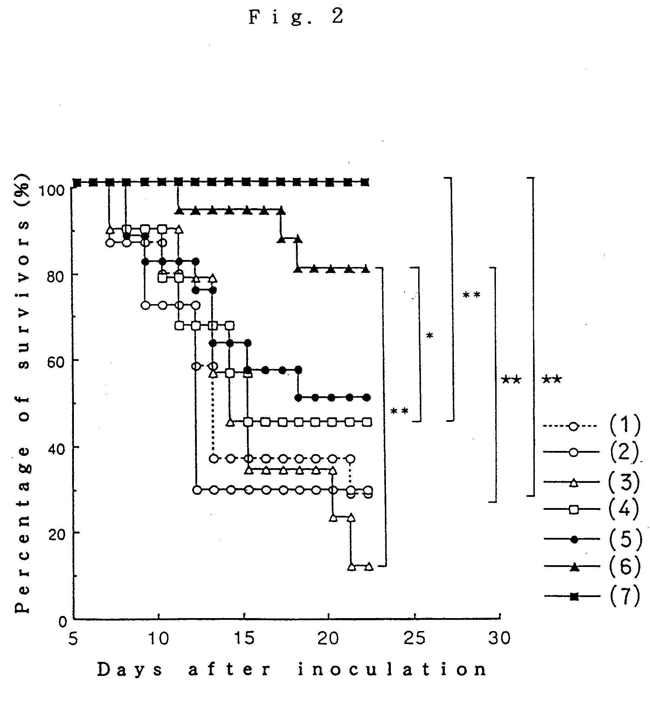

For the same groups as the groups tested above in a), the results of survival rate analysis are shown in Fig. 2 (ordinate: percent survival rate; abscissa: number of days after inoculation) using the same legends as used in Fig. 1.

As seen in Fig. 2, the groups showing a higher survival rate value than that obtained in the infected control group (graph (1), 28.6%) among the groups intratracheally given m-γIFN were as follows: 100 IU m-γIFN group (graph (5), 50%), 1,000 IU m-γIFN group (graph (6), 80%) and 10,000 IU m-γIFN group (graph (7), 100%).

Furthermore, dose dependence was observed in the m-γIFN groups at the 1% level of significance (p = 0.0006). A life span-prolonging effect was observed in the 1,000 IU group and 10,000 IU group at the 1% level of significance (★★: p = 0.0093 and p = 0.0012, respectively).

The difference in survival rate between the 1,000 IU m-γIFN group (graph (6)) and the 1,000 IU m-α/βIFN group (graph (3)) was significant at the 1% level (**: p = 0.0042) and the difference between the 1,000 IU m-γIFN group (graph (6)) and the 10,000 IU m-α/βIFN group (graph (4)) was significant at the 5% level (*: p = 0.0225). The difference between the 10,000 IU m-γIFN group (graph (7)) and the 10,000 IU m-α/βIFN group (graph (4)) was significant at the 1% level (**: p = 0.0015). Thus, each comparison revealed the life span-prolonging effect in each m-γIFN group.

The above results clearly indicate that the m-γIFN groups show a significant life span prolonging effect as com-

pared with the m-$\alpha/\beta$IFN groups.

c) Endocardial viral replication inhibiting effect

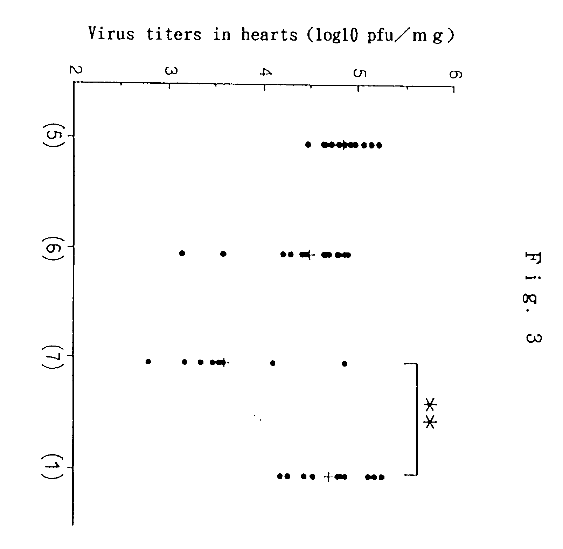

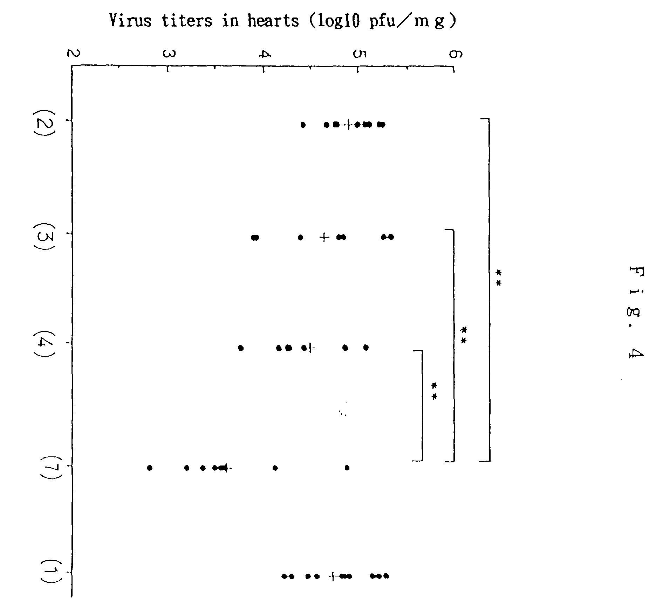

The results of testing for endocardial viral replication inhibiting effect are shown in Fig. 3 and Fig. 4 (ordinate: endocardial virus titer; abscissa: each dosed group).

As is clear from the results shown in Fig. 3, the endocardial viral replication was significantly inhibited in the group intratracheally given 10,000 IU/mouse of m-$\gamma$IFN (graph (7)) ($3.6 \pm 0.2$ $\log_{10}$ pfu/mg) as compared with the infected control group (graph (1)) ($4.9 \pm 0.2$ $\log_{10}$ pfu/mg) at the 1% level.

Furthermore, the 10,000 IU/mouse m-$\gamma$IFN group (graph (7)), when compared with the m-$\alpha/\beta$IFN groups (graphs (2), (3) and (4) shown in Fig. 4), inhibited the endocardial viral replication significantly at the 1% level.

d) Cardiac pathologic picture improving effect

1. Ventricular wall lesion

In the m-$\gamma$IFN groups, a significant dose dependence was noted at the 1% level (p = 0.0006). Furthermore, the ventricular wall lesion was significantly inhibited in the 10,000 IU/mouse m-$\gamma$IFN group (lesion score: $+3.3 \pm 0.8$) at the 5% level (p = 0.0161) as compared with the mouse ventricles in the infected control group (lesion score: $+3.9 \pm 0.3$).

2. Necrosis of myocardial cells

In the m-$\gamma$IFN groups, a significant dose dependence was noted at the 1% level (p = 0.0016). Furthermore, the necrosis of ventricular cells was significantly inhibited in the group intratracheally given 10,000 IU/mouse of m-$\gamma$IFN (lesion score: $+2.4 \pm 0.7$) at the 5% level (p = 0.0170) as compared with the mouse ventricles in the infected control group (lesion score: $+2.9 \pm 0.3$).

3. Infiltration of inflammatory cells

No significant difference was observed between the groups intratracheally given m-$\gamma$IFN and the infected control group.

4. Formation of calcification foci

In the groups intratracheally given m-$\gamma$IFN, a significant dose dependence was observed at the 5% level (p = 0.0164). Furthermore, the calcification focus formation on the ventricular wall was significantly inhibited in all the groups intratracheally given m-$\gamma$IFN (100 IU/mouse group, lesion score: $+2.0 \pm 0.4$; 1,000 IU/mouse group, lesion score: $+2.1 \pm 0.5$; 10,000 IU/mouse group, lesion score: $+1.9 \pm 0.6$) at the 1% level (p = 0.0001, p = 0.0004 and p = 0.0002, respectively) as compared with the mouse ventricles in the infected control group (lesion score: $+2.9 \pm 0.3$).

5. Fibrosis of ventricular wall

None of the groups inclusive of the infected control group showed fibrosis of the ventricular wall.

The foregoing results are definite evidence of the life span-prolonging effect, cardiac pathology improving effect and endocardial virus replication inhibitory effect of $\gamma$-IFN aministered intratracheally in the subacute coxsackievirus B myocarditis mouse model. Particularly, the survival rate, life span-prolonging effect and endocardial virus replication inhibiting effect were unexpectedly and significantly superior even when compared with $\alpha$-/$\beta$-IFN.

<u>Example 2</u> Effect of combined use of $\gamma$-IFN in the coxsackievirus B myocarditis model

(1) Construction of a viral myocarditis model

A viral myocarditis model was constructed in the same manner as in Example 1 by intraperitoneally inoculating 3-week-old C3H/He slc mice with a viral dilution derived from a strain obtained by subculturing the Nancy strain of coxsackievirus B3 in Hela (3 generations) and C3H/He mice (12 generations).

(2) Preparation of test drugs

A murine γ-IFN sample was prepared by diluting m-γIFN (Hayashibara Biochemical Laboratories, Okayama Prefecture; lot number: 004003; activity: $4.74 \times 10^5$ IU/ml) to $1 \times 10^5$ IU/ml using physiological saline and further adjusted to $1 \times 10^2$ IU/ml using 0.01% mouse albumin-physiological saline (0.01% mAlb).

Murine α- and β-IFN samples were prepared by diluting m-α/βIFN (Hayashibara Biochemical Laboratories, Okayama Prefecture; lot number: 005; activity: $4.96 \times 10^7$ IU/ml) to $1 \times 10^3$ and $1 \times 10^4$ IU/ml using 0.01% mAlb).

(3) Administration of each IFN to virus-infected mice

Model mice produced in (1) were intramuscularly dosed with the respective drug solutions prepared in (2) either singly or in combination or with the control preparation mentioned below for 4 consecutive days after virus inoculation.

Group 1  - infected control group (given 0.01% mAlb)
Group 2  - group given m-γIFN (10 IU/mouse) alone
Group 3  - group given m-α/βIFN (1,000 IU/mouse) alone
Group 3'  - group given m-α/βIFN (100 IU/mouse) alone
Group 4  - group combinedly given m-γIFN and m-α/βIFN (1,000 IU/mouse)
Group 4'  - group combinedly given m-γIFN and m-α/βIFN (100 IU/mouse)

(4) Investigation of effects

The increases in survival rate, the endocardial viral replication inhibiting effects and the cardiac picture improving effects attained in the test were determined and analyzed in the same manner as in Example 1.

Endocardial viral counts were determined as follows. Seven days after inoculation, the heart was excised aseptically, washed with physiological saline and processed into a 10% emulsion (10% E-MEM 0.1 ml/heart) using Polytron (Kinematica AG, model K). The supernatant derived from the emulsion (3000 rpm, 4°C, for 10 minutes) was serially diluted (10 levels) with 10% E-MEM. FL monolayer cells were inoculated with 0.1 ml of each dilution and submitted to plaque assay. After incubation at 37°C for 4 days in a 5% carbon dioxide gas incubator, the layered medium was discarded, a 10% buffered formalin solution supplemented with 0.05% crystal violet was distributed in 1-ml portions and fixation and staining were effected overnight at room temperature. On the next day, the staining solution was discarded, the plate was washed with physiological saline and dried, and then plaques were counted. The virus count (pfu/mg, detection limit < 1 pfu/mg heart) was calculated by multiplying the number of plaques by the dilution factor. For statistical analysis, one-way analysis of variance was carried out and then Tukey test was performed for multiple comparison. The 5% level of significance was used in the evaluation.

Pathological sections of each heart excised 10 days after inoculation with the virus were used as samples for pathological evaluation of the heart.

Furthermore, according to the report of Connell et al. [Connel, E. V., et al., 1985, in The Biology of Interferon System; pp. 419-422 and Aapro, M. S., et al., 1983, Cancer Chemother. Pharmacol., 10, 161-166] using encephalomyocarditis virus and other virus infection models, the effect of the combined use of m-γIFN and m-α/βIFN was examined by determining the death or survival of each mouse 21 days after inoculation and judging whether said effect corresponded to synergism, additivity or antagonism (see Table 1 given below).

Table 1

| Synergism | FMa + b < FMa x FMb |
|---|---|
| Additivity | FMa + b = FMa x FMb |
| Antagonism | FMa + b > FMa x FMb |
| FM: Mortality; a, b: each mIFN; a+b: combined use | |

(6) Results

The results obtained are shown below, item by item.

a) Survival rate and effect of combined use

As shown below in Table 2, some increase in survival rate was observed in the combination (two-agent) treatment group (group 4) as compared with each IFN single-agent treatment group (group 2 or group 3). This combination-derived effect was suggested to be a synergism.

Table 2

|  | Mortality (FM) | FMa x FMb | Effect of combined use |
|---|---|---|---|
| Group 2 (a) | 19/20 (0.95) | - | - |
| Group 3 (b) | 18/20 (0.90) | - | - |
| Group 4 (a+b) | 15/20 (0.75) | 0.86 | Synergistic |

b) Endocardial viral replication inhibiting effect

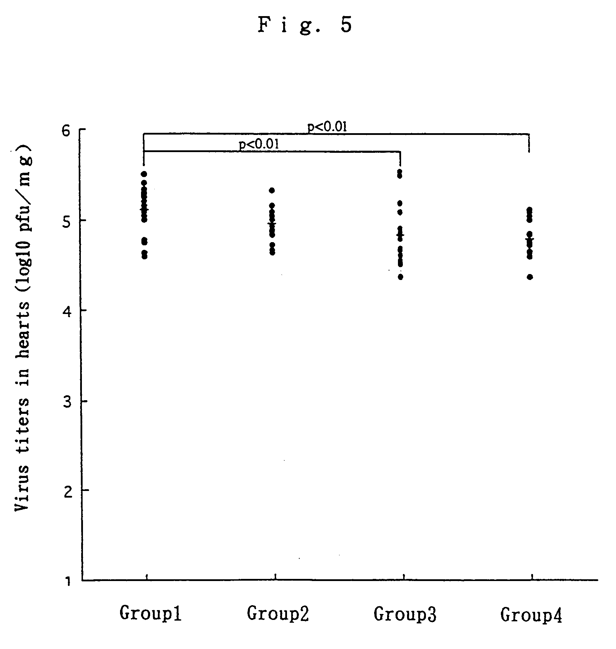

The results are shown in Fig. 5.
In the combined use (two-agent) group (Group 4, n = 19), the endocardial virus count was $4.80 \pm 0.2 \log_{10}$ pfu/mg.
As compared with the value ($5.1 \pm 0.3 \log_{10}$ pfu/mg) in the infected control group (group 1, n = 20), this value was judged as indicative of a significant ($p<0.01$) endocardial viral replication inhibiting effect.
In the m-$\alpha/\beta$IFN single-agent treatment group (group 3, n = 19), too, substantially the same effect was observed.

c) Histopathological examination

c-1) Inflammatory cell infiltration

The results are shown in Table 3. Each of the tests described below was performed in 5 groups, with an uninfected and non-treated group additionally provided.

Table 3

|  | Group 5 | Group 1 | Group 2 | Group 3 | Group 4 |
|---|---|---|---|---|---|
| n = | 10 | 19 | 19 | 20 | 18 |
| Score |  |  |  |  |  |
| 0 | 10 | 0 | 0 | 0 | 0 |
| +1 | 0 | 0 | 1 | 0 | 5 |
| +2 | 0 | 8 | 15 | 6 | 8 |
| +3 | 0 | 11 | 3 | 14 | 5 |
| +4 | 0 | 0 | 0 | 0 | 0 |
| Total | 0 | 49 | 40 | 54 | 36 |
| Index | 0 | 2.6 | 2.1 | 2.7 | 2.0 |
| Test* | - | - | 0.0066 | NS | 0.0183 |

Test* = Mann-Whitney U test (provided, however, that Bonferroni's correction was made to adjust for multiplicity). Lesion scores were arrived at as follows. According to the percentage of the lesion based on the total area of the heart (ventricular) section, the case in which the percentage of the lesion was less than 25% was graded as +1; 25 to 50% as +2; 50 to 75% as +3; and 75 to 100% as +4.

It is apparent from Table 3 that compared with the infected control group (Group 1), the two-agent treatment group (Group 4) showed a significant inhibition of inflammatory cell infiltration at the 5% level (p = 0.0183). Furthermore, compared with the m-$\alpha/\beta$IFN single-agent treatment group (Group 3), the inhibitory effect obtained in Group 4 was significant at the 1% level (p = 0.0035). However, the effect obtained in Group 4 was slight and not significant as compared with the m-γIFN single-agent treatment group (Group 2).

c-2) Inflammatory cell infiltration

In lieu of Group 3 (m-α/βIFN 1,000 IU/mouse single-agent treatment group), a group receiving m-α/βIFN 100 IU/mouse (n=19) was established as Group 3'. Then, the above test was repeated and the necrosis of heart muscle cells was investigated and analyzed statistically. The results are set forth in Table 4.

Table 4

|  | Group 5 | Group 1 | Group 2 | Group 3' | Group 4' |
|---|---|---|---|---|---|
| n = | 10 | 19 | 18 | 19 | 17 |
| Score |  |  |  |  |  |
| 0 | 10 | 0 | 0 | 0 | 0 |
| +1 | 0 | 1 | 0 | 1 | 5 |
| +2 | 0 | 6 | 9 | 7 | 11 |
| +3 | 0 | 12 | 9 | 11 | 1 |
| +4 | 0 | 0 | 0 | 0 | 0 |
| Total | 0 | 49 | 45 | 48 | 30 |
| Index | 0 | 2.6 | 2.5 | 2.5 | 1.8 |
| Test* | - | - | NS | NS | 0.0015 |

Test* = Mann-Whitney U test (provided, however, that Bonferroni's correction was made to adjust for multiplicity). Lesion scores were arrived at as follows. According to the percentage of the lesion based on the total area of the heart (ventricular) section, the case in which the percentage of the lesion was less than 25% was graded as +1; 25 to 50% as +2; 50 to 75% as +3; and 75 to 100% as +4.

It is apparent from Table 4, too, that compared with the infected control group (Group 1), the two-agent treatment group (Group 4') showed a significant inhibition of inflammatory cell infiltration at the 1% level (p = 0.0015). Furthermore, compared with either mIFN single-agent treatment group (Group 2 and Group 3'), this Group 4' showed a significant inhibition of inflammatory cell infiltration at the 1% level (p = 0.0045 and p = 0.0045).

c-3) Necrosis of heart muscle cells

The results of observation and analysis of the necrosis of heart muscle cells in the above test using Group 3' are presented in Table 5.

Table 5

| | Group 5 | Group 1 | Group 2 | Group 3' | Group 4' |
|---|---|---|---|---|---|
| n = | 10 | 19 | 18 | 19 | 17 |
| Score | | | | | |
| 0 | 10 | 0 | 0 | 0 | 0 |
| +1 | 0 | 0 | 1 | 1 | 4 |
| +2 | 0 | 4 | 2 | 2 | 10 |
| +3 | 0 | 15 | 15 | 16 | 3 |
| +4 | 0 | 0 | 0 | 0 | 0 |
| Total | 0 | 53 | 50 | 53 | 33 |
| Index | 0 | 2.8 | 2.8 | 2.8 | 1.9 |
| Test* | - | - | NS | NS | 0.0006 |

Test* = Mann-Whitney U test (provided, however, that Bonferroni's correction was made to adjust for multiplicity). Lesion scores were arrived at as follows. According to the percentage of the lesion based on the total area of the heart (ventricular) section, the case in which the percentage of the lesion was less than 25% was graded as +1; 25 to 50% as +2; 50 to 75% as +3; and 75 to 100% as +4.

It is apparent from Table 5 that compared with the infected control group (Group 1), the two-agent treatment group (Group 4') showed a significant inhibition of myocardial cell necrosis at the 1% level (p = 0.0006). Moreover, compared with either mIFN single-agent treatment group (Group 2 and Group 3'), this Group 4' showed a significant inhibition of heart muscle cells at the 1% level (p = 0.0015 and p = 0.0010).

The foregoing results suggested that the survival rate-increasing effect of the combination treatment with 1,000 IU/mouse of m-$\alpha$/$\beta$IFN and 10 IU/mouse of m-$\gamma$IFN is derived from the antiviral effect provided by 1,000 IU/mouse of m-$\alpha$/$\beta$IFN and the inhibitory effect of 10 IU/mouse of m-$\gamma$IFN on inflammatory cell infiltration.

Moreover, the effect of the combination treatment with 100 IU/mouse of m-$\alpha$/$\beta$IFN and 10 IU/mouse of m-$\gamma$IFN, that is to say the inhibitory effect on heart muscle cell necrosis and inflammatory cell infiltration, is a synergistic effect of the two drug substances.

The following are preparation examples of the therapeutic composition for coxsackievirus B infection of the invention. In these examples, the following agents were used.

1) IFN-$\gamma$ standard solution (manufactured by Hayashibara Biochemical Laboratories):

A standard solution wherein IFN derived from human HBL-38 cells (specific activity of 10000000 IU/mg) is dissolved in an amount of 1000000 IU/ml in a phosphate buffer (pH 7.2) containing 0.1 w/v% of human serum albumin as a stabilizer.

2) IFN-$\alpha$ standard solution (manufactured by Hayashibara Biochemical Laboratories):

A standard solution wherein IFN derived from human BALL-1 cells (specific activity of 50000000 IU/mg) is dissolved in an amount of 5000000 IU/ml in a phosphate buffer (pH 6) containing 0.1 w/v% of human serum albumin as a stabilizer.

3) Sucrose fatty acid esters:

Sucrose lauric acid ester, sucrose palmitic acid ester, sucrose stearic acid ester and sucrose oleic acid ester (all are manufactured by Mitsubishi Chemical Food Co.,).

Preparation Example 1

A 50 mg quantity of sucrose lauric acid ester was dissolved in 1 ml of IFN-γ standard solution, and D-mannitol was added thereto. The mixture was adjusted to osmotic ratio of 2 and lyophilized to afford a inhalant preparation according to the present invention.

This preparation is given as dissolved in 10 ml of water prior to use.

Preparation Example 2

A 50 mg quantity of sucrose lauric acid ester was added to IFNs solution, which contain 1 ml of IFN-γ standard solution and 2 ml of IFN-α standard solution, followed by isotonization with D-mannitol and the resultant mixture was lyophilized to afford a inhalant preparation of the present invention.

This preparation is given as dissolved in 10 ml of water prior to use.

**Claims**

1.  A therapeutic composition for coxsackievirus B infection which comprises gamma-interferon as an active ingredient.

2.  A therapeutic composition for coxsackievirus B infection as claimed in Claim 1, wherein the gamma-interferon is selected from among natural gamma-interferon, genetically engineered gamma-interferon and a gamma-interferon derivative having gamma-interferon activity.

3.  A therapeutic composition for coxsackievirus B infection as claimed in Claim 1, wherein the coxsackievirus B infection occurs in the myocardium and/or pericardium.

4.  A therapeutic composition for coxsackievirus B infection as claimed in Claim 2, wherein the coxsackievirus B infection occurs in the myocardium and/or pericardium.

5.  A therapeutic composition for coxsackievirus B infection as claimed in any of Claims 1 to 4, wherein the coxsackievirus B infection is coxsackievirus B3 infection.

Fig. 1

Fig. 2

Fig. 3

Fig. 4

F i g. 5

## INTERNATIONAL SEARCH REPORT

| | International application No. |
|---|---|
| | PCT/JP96/02064 |

**A. CLASSIFICATION OF SUBJECT MATTER**

Int. Cl$^6$  A61K38/21

According to International Patent Classification (IPC) or to both national classification and IPC

**B. FIELDS SEARCHED**

Minimum documentation searched (classification system followed by classification symbols)

Int. Cl$^6$  A61K38/21

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| P | Chemical Abstracts, Vol. 124 (1996), Abstract No. 314891 (J. Interferon Cytokine Res., Vol. 16, No. 4 (1996), p. 283-287, (A. Heim et al.)) | 1 - 5 |
| Y | Chemical Abstracts, Vol. 122 (1995), Abstract No. 45760 (Lett. Appl. Microbiol., Vol. 19, No. 5 (1994), p. 386-390, (K. Konishi et al.)) | 1 - 5 |
| Y | Chemical Abstracts, Vol. 118 (1993), Abstract No. 100060 (J. Infect. Dis., Vol. 166, No. 5 (1992), p. 958-965, (A. Heim et al.)) | 1 - 5 |
| Y | Chemical Abstracts, Vol. 110 (1989), Abstract No. 88192 (Antiviral Res., Vol. 10 (1988) p. 129-140, (N. G. Ilbaeck et al.), | 1 - 5 |
| A | JP, 6-298665, A (Toray Industries, Inc.), October 25, 1994 (25. 10. 94) (Family: none) | 1 - 5 |

| X | Further documents are listed in the continuation of Box C. | | See patent family annex. |
|---|---|---|---|

\* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| September 25, 1996 (25. 09. 96) | October 8, 1996 (08. 10. 96) |

| Name and mailing address of the ISA/ | Authorized officer |
|---|---|
| Japanese Patent Office | |
| Facsimile No. | Telephone No. |

Form PCT/ISA/210 (second sheet) (July 1992)